# FASCICULE DE BREVET EUROPEEN

(11) **EP 3 468 519 B1**
(45) Date de publication et mention de la délivrance du brevet: **17.06.2020**
(21) Numéro de dépôt: 15820882.7
(22) Date de dépôt: 09.11.2015
(51) Int. Cl.: A61G 13/00, A61G 13/10, A61G 13/12, A61B 6/04, A61B 8/00, A61B 5/08, A63B 22/00, A61G 13/04, A61G 13/06, A61G 13/08, A61B 6/00, A61B 8/08, A61B 8/12, A63B 22/06

(54) **PLATEAU D'EXAMEN POLYVALENT ADAPTÉ AUX ÉCHOCARDIOGRAPHIES**
MEHRZWECKUNTERSUCHUNGSTISCH FÜR DIE ECHOKARDIOGRAPHIE
MULTI-PURPOSE EXAMINATION TABLE SUITABLE FOR ECHOCARDIOGRAPHY

(43) Date de publication de la demande: 17.04.2019
(73) Titulaire: Koch, Michel, 33200 Bordeaux (FR)
(72) Inventeur: Koch, Michel, 33200 Bordeaux (FR)
(86) Numéro de dépôt international: PCT/IB2015/058639
(87) Numéro de publication internationale: WO 2017/077369

(56) Documents cités:
- DE-A1-102006 056 282
- US-A- 5 018 724
- US-A- 5 678 263
- US-A1- 2003 114 275
- US-A1- 2015 038 826

## Description

La présente invention concerne un dispositif de plateau d'examen polyvalent, adapté à l'échocardiographie de stress à l'effort ou pharmacologique, à l'échocardiographie de repos, à l'échocardiographie transoesophagienne, à l'angiologie et aux examens de repos, permettant de passer dans un temps très court d'une séquence d'examen à une autre séquence d'examen, de la manière la plus rapide possible et dans des conditions de confort, de rapidité et de qualité d'examens améliorées.

Dans l'état actuel du marché, pour les examens d'échocardiographie, il est courant de trouver des tables d'examen d'échocardiographie de repos, conçues pour répondre aux examens conventionnels à l'aide d'ultra sons et permettant d'observer et d'analyser le cœur et les vaisseaux. Le patient est allongé et l'examen se fait, pour la partie cardiologique, au travers du thorax et selon un angle souhaité, pour détecter des anomalies. On demande aussi au patient de se tourner sur le coté gauche coude relevé pour ouvrir la « fenêtre » ultrasonore entre deux côtes en plaquant le cœur contre la paroi. Cependant, de nombreux patients ont des difficultés physiques à s'adapter à cette position (personnes âgées, obèses, post opératoires etc...). Certaines tables d'examen sont ouvertes sur le côté gauche du flanc pour mieux accéder à la pointe du coeur, mais cette disposition est insuffisante pour procéder à des examens complets du coeur et n'améliore pas le contact cœur-paroi. L'apparition sur le marché de tables d'examen telles que connue de US 2003/114275 A1, pourvues d'un ergomètre pour procéder à l'échocardiographie de stress et pour analyser la fonction cardiaque du ventricule au cours de l'effort, permet d'améliorer les connaissances sur l'état cardiaque du patient. Cependant, les procédures sont longues et inconfortables pour le patient. Par ailleurs, ces tables ne permettent pas de pratiquer intégralement l'ensemble des examens d'échocardiographie et d'angiologie, aujourd'hui nécessaires dans une même salle pour différents patients et dans un temps très court. Elles sont par ailleurs difficiles à manoeuvrer.

Le dispositif selon l'invention permet de remédier à ces inconvénients et d'améliorer les performances d'ensemble des examens du coeur.

Il comporte en effet, un plateau technique constitué d'un matelas en matière semi dure de type médical, composé de deux parties, l'une recevant la tête et le buste, l'autre partie recevant le fessier et les membres inférieurs.

Selon une première caractéristique, la morphologie de la têtière est volontairement asymétrique pour permettre le rapprochement de l'appareil échographe nécessaire au repos, mais encore plus, en séquence d'effort et lors de la montée-descente du plateau. Cette disposition permet une amélioration du confort échographiste, une meilleure qualité d'examen et évite la détérioration de l'échographe onéreux. Dans un axe longitudinal de positionnement du patient, la partie têtière du plateau comporte un orifice de forme oblongue, adapté au calage de la tête du patient. Cette forme oblongue a pour intérêt de favoriser l'hyper extension du cou, pour un examen échographique plus précis des artères du cou. La forme de cette orifice et la distance au bord supérieur de la têtière, sont le fruit d'observations cliniques. Hors cet examen, l'orifice est comblé par une pièce adaptée à sa forme , pour reproduire une surface unie et pleine.

Selon une seconde caractéristique, la partie têtière du plateau comporte du côté cœur, un support à trois positions variables : position un : prolongement de la têtière . Position deux : calage du bord externe de l'épaule chez les patients à limitations articulaires. Position trois : soutien d'aisselle si le patient peut lever le coude. Ce support permet de dégager l'épaule et le bras, à fin d'ouvrir plus largement la zone côté cœur du thorax, dans le but d'un examen d'échocardiographie plus précis. Hors cet examen, la mobilité du support permet de dégager la surface de la têtière pour un examen conventionnel du patient reposant sur le dos ou sur son côté gauche. Dans cette configuration, les extrémités basses et hautes de ce support de calage de hanche, délimitent une ouverture latérale d'accès à la pointe du cœur.

Selon une troisième caractéristique, la partie du plateau destinée à recevoir le fessier et les jambes comporte un appui latéral apparaissant automatiquement à l'inclinaison pour le calage de la hanche, lorsque ce plateau est latéralement incliné côté cœur. Cet appui est constitué de deux cylindres en matière semi dure, disposés l'un au dessus de l'autre et montés libres sur un cadre en forme de « U », fixé sur le plateau. Cet appui est fixe alors que le plateau est mobile, d'où son apparition automatique. Deux cylindres sont nécessaires pour constituer un appui suffisamment haut et efficace lors de l'inclinaison du patient sur le côté gauche. Les cylindres montés libres sur chaque axe, permettent d'éviter les pincements de l'épiderme. Le système fait également office de soutien du coude et prévient les lésions de son membre supérieur et de sa colonne vertébrale en optimisant sa posture (prévention des maladies professionnelles).

Selon une quatrième caractéristique, le plateau dispose d'un support pour adapter un ergomètre et ses accessoires, utilisé pour la réalisation d'une échocardiographie de stress à l'effort. Cet ergomètre prend appui sur un axe mobile transversal, situé à l'extrémité du plateau et dans le prolongement des jambes. Cet axe mobile présente l'avantage de pouvoir relever l'ergomètre sous un angle à plus de 180 degrés, pour dégager la surface du plateau par l'intermédiaire d'un système motorisé, en dehors des séquences d'échocardiographie de stress, sans que cet appareil constitue une contrainte à la position basse du plateau. Autre avantage: l'ergomètre situé au dessus du plateau peut être ajusté au pédalage du patient avant l'effort, au moyen d'un réglage coulissant, puis pendant l'effort au moyen du même système de relèvement utilisé pour l'escamoter.

Selon une cinquième caractéristique, le plateau dispose d'un appui fessier en matière semi dure. Ce dispositif permet d'éviter l'utilisation d'une selle inconfortable et inadaptée à toutes les morphologies Cet appui fessier permet au patient de se caler sur le plateau à fin d'utiliser l'ergomètre, de la manière la plus performante et la plus confortable possibles, en vue d'un examen d'échocardiographie le plus représentatif de son état. En dehors des séquences d'échocardiographie de stress à l'effort, l'appui fessier est escamoté; sa surface est en continuité avec la surface du plateau.

Selon une sixième caractéristique, le plateau dispose d'une poignée de maintien latérale réversible et escamotable, située sur le côté droit du corps. Cette poignée est à double fonction: la première est de permettre au patient de disposer d'un appui lors des séquences d'échocardiographie d'effort; escamoté, le plateau présente une échancrure permettant au patient de parfaitement positionner ses fesses tant pour les examens de repos que d'effort, et à l'échocardiographiste, de s'approcher du patient. Ainsi, le système est parfaitement adapté pour un travail à gauche comme à droite du patient. La seconde fonction est de servir de support d'avant-bras du patient pour poser la perfusion lors des échocardiographies de stress pharmacologiques ou d'échographies transoesophagiennes et de laisser ensuite le bras tendu. Une attache souple peut compléter le dispositif pour éviter que le patient bouge son bras durant l'examen.

Selon une septième caractéristique, non seulement le plateau dispose des possibilités courantes aux tables de soins de pouvoir pivoter et s'incliner transversalement et longitudinalement de la tête vers les pieds, en particulier, pour la séquence d'examen d'échocardiographie d'effort qui nécessite un proclive suffisant pour pédaler, mais il présente l'avantage de pouvoir s'incliner à l'inverse, des pieds vers la tête (position Trendelenburg), pour traiter le malaise vagal pouvant survenir chez des patients durant cette séquence. Le malaise vagal se résout en général spontanément et rapidement, en élevant les jambes du patient.

La motorisation de toutes les opérations d'adaptation du plateau, à toutes les séquences d'examens, permet un gain sensible de temps dans le déroulement de ces séquences ainsi qu'une moindre fatigue pour les opérateurs, un plus grand confort pour les patients et des examens de meilleure qualité et plus rapides en diminuant les temps de transfert. La possibilité d'angulation de la têtière à 80 degrés de l'horizontal, puis de la rabaisser, le passage en déclive latéral gauche, le tout par systèmes télécommandés, permet d'améliorer la technique d'échocardiographie transoesophagienne. Le patient peut avaler la sonde en position assise plutôt que couché sur le côté, ce qui est plus aisé pour lui. La manœuvre électrique permet ensuite de rabaisser son buste et de le tourner sur le côté gauche pour éviter les fausses routes.

Le dispositif selon l'invention est particulièrement destiné aux examens d'échocardiographie de repos, d'échocardiographie transoesophagienne, d'échocardiographie de stress et d'angiologie.

Les dessins annexés illustrent l'invention :

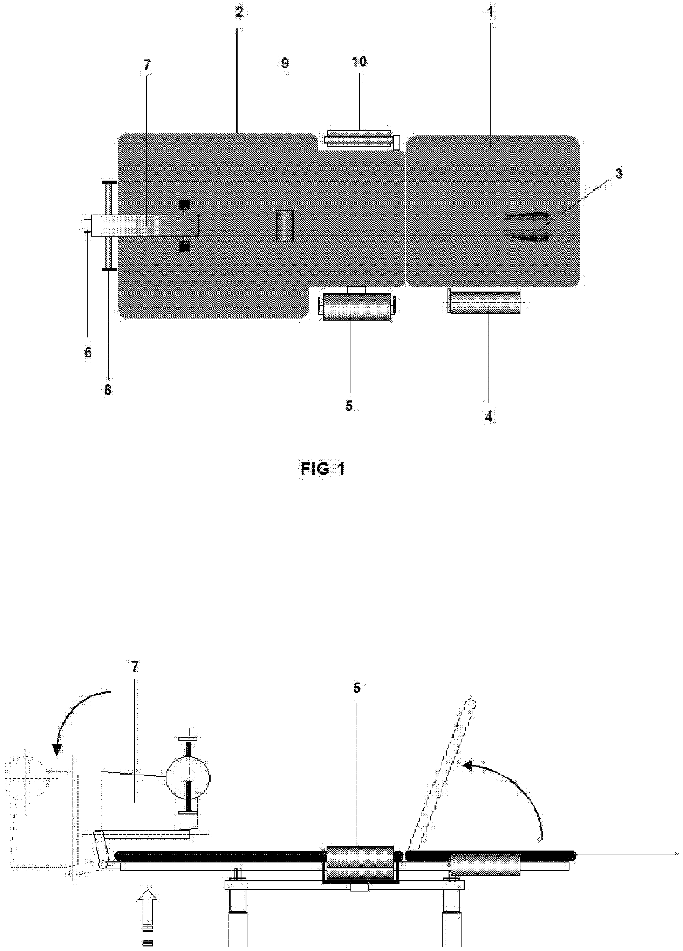

La figure 1 représente le dispositif selon de l'invention vu en plan.
La figure 2 représente le dispositif selon l'invention vu en élévation en position de repos.

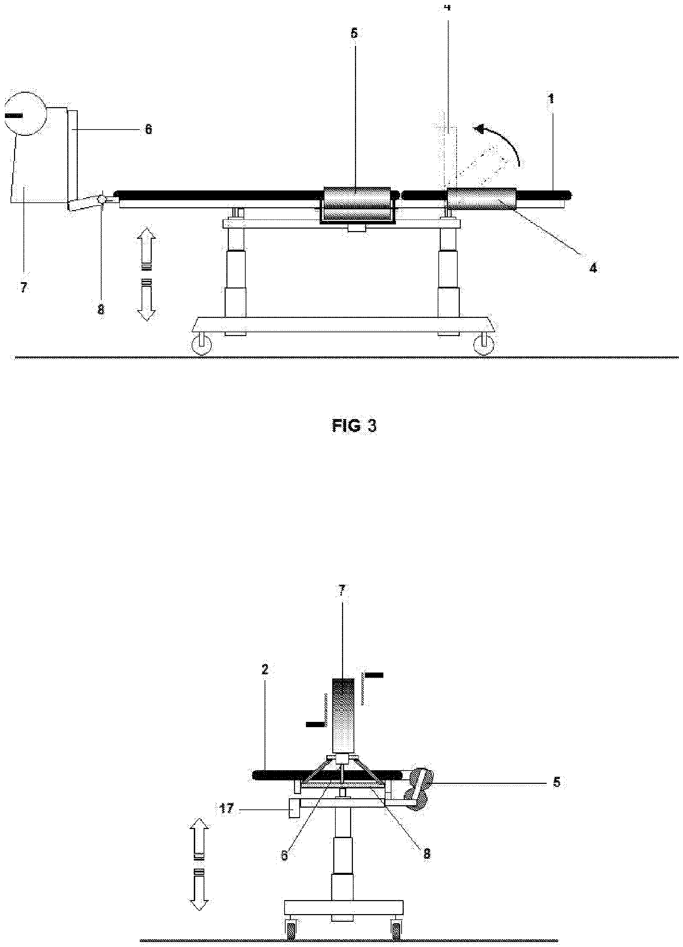

La figure 3 représente le dispositif selon l'invention vu en élévation et son support à trois positions sur le côté cœur de la têtière.
La figure 4 représente le dispositif selon l'invention vu en élévation sur le petit côté pour les examens de repos.

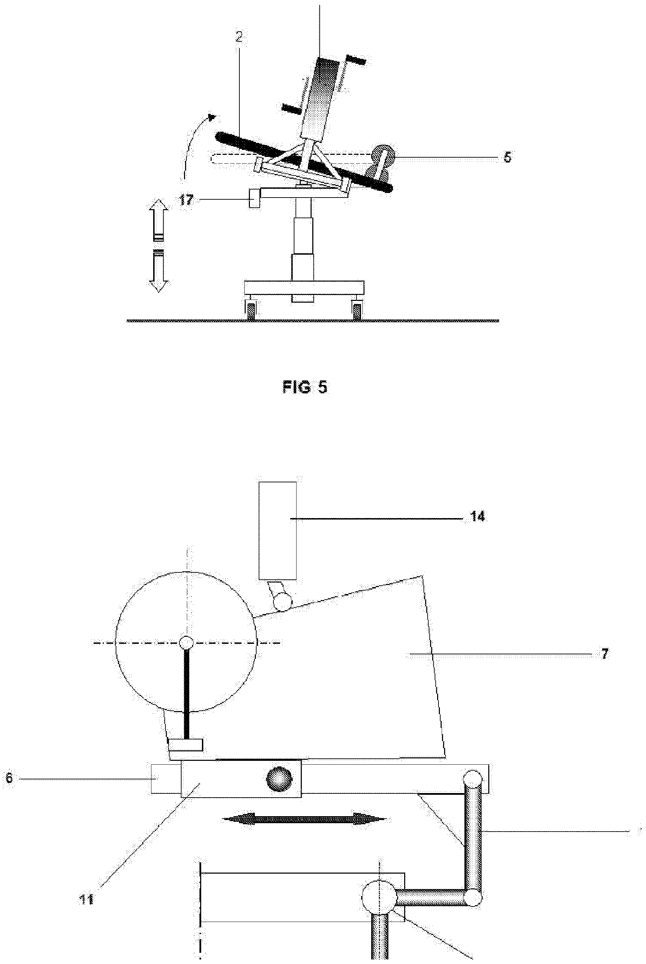

La figure 5 représente le dispositif selon l'invention vu en élévation sur le petit côté pour les examens de stress à l'effort.
La figure 6 représente une vue en élévation du support de l'ergomètre

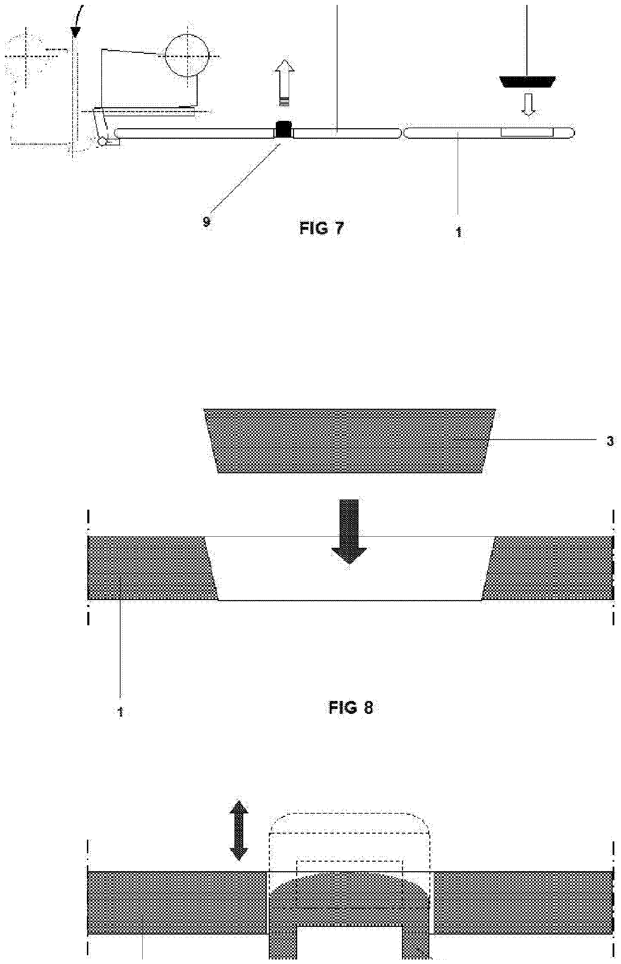

La figure 7 représente le plateau du dispositif selon l'invention vu en coupe longitudinale.
La figure 8 représente une vue en coupe du détail de l'orifice adapté à la tête du patient.
La figure 9 représente une vue en coupe du détail de l'appui fessier

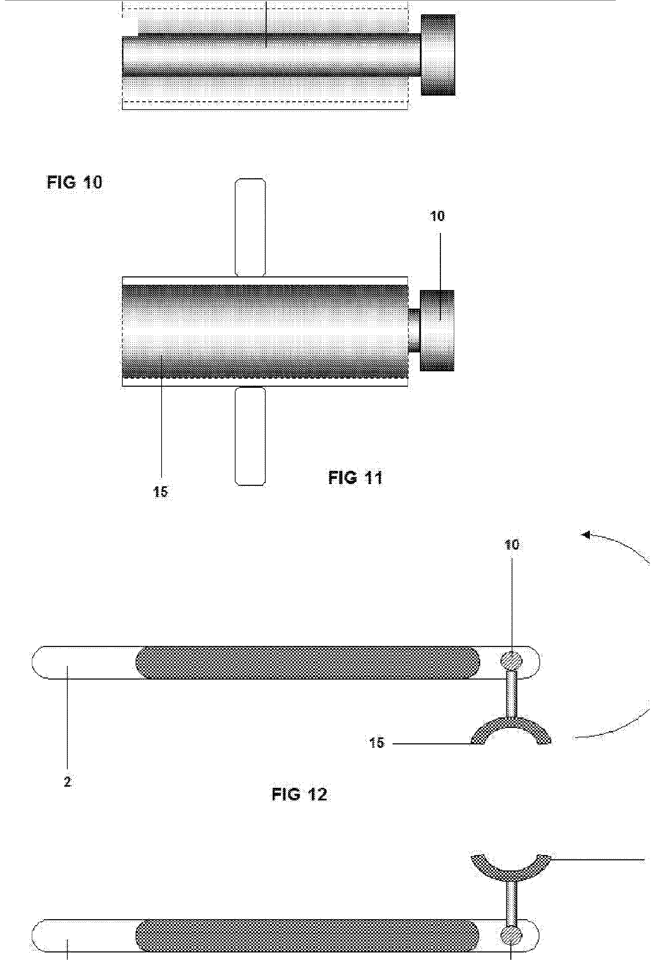

La figure 10 représente une vue en plan du détail de la poignée de maintien latérale.
La figure 11 représente une vue en plan du détail du support de l'avant bras lors de perfusions.
La figure 12 représente une section du plateau d'examens avec la poignée de maintien latérale côté droit.
La figure 13 représente une section du plateau d'examens avec le support de l'avant bras côté droit.

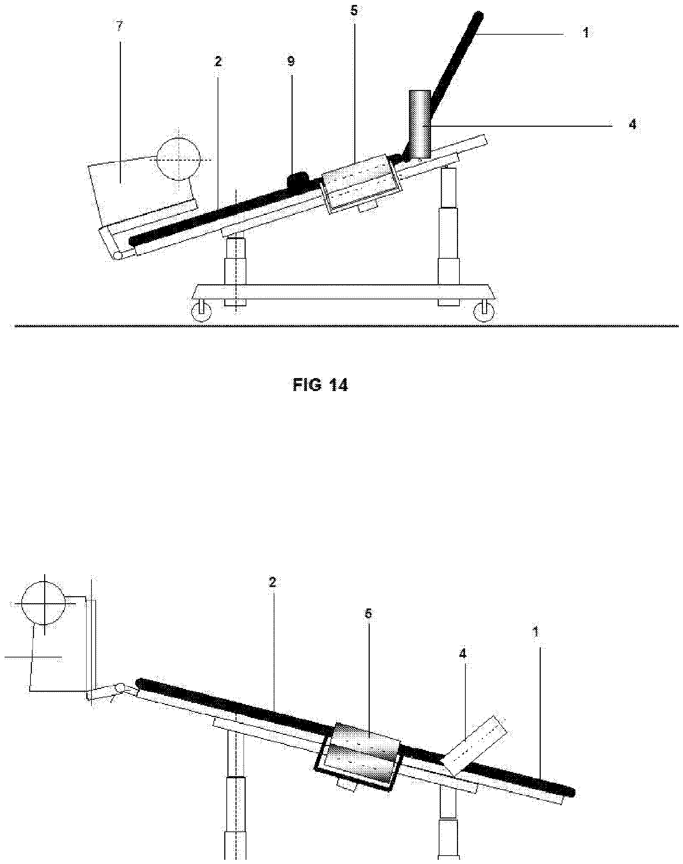

La figure 14 représente le dispositif selon l'invention vu en élévation adapté aux examens de stress à l'effort sans inclinaison côté cœur.
La figure 15 représente le dispositif selon l'invention vu en élévation adapté au traitement du malaise vagal.

En référence à ces dessins, le dispositif comporte un plateau d'examen composé de 2 parties: une partie têtière et une partie destinée au fessier et aux membres inférieurs:
Sur la partie têtière (1) : un orifice (3) de forme oblongue adapté au calage de la tête pouvant être comblé par une pièce de forme identique, côté cœur un support de forme cylindrique (4) à trois positions.
Sur la partie destinée à recevoir le fessier et les membres inférieurs du plateau (2), un appui (5) composé de deux cylindres montés verticalement et inclinés vers l'extérieur. Cet appui est fixé sur le châssis du plateau (2). Un support (6) permettant la réception d'un ergomètre (7) et de ses accessoires. Ce support prend appui sur un axe transversal (8) monté sur le châssis du plateau (2) et situé à son extrémité . Un appui fessier (9) de forme rectangulaire et bombée sur le dessus. Une poignée latérale réversible (10) fixée sur le côté droit du châssis du plateau (2), un support de boîtiers de commandes (17) fixé sur le châssis du plateau (2).

Selon un mode particulier de réalisation, le support (6) de l'ergomètre (7) avec ses accessoires (14) est monté sur un axe libre (8) par l'intermédiaire d'un bras (12) de façon que ce support se situe au dessus du plateau (2). L'ergomètre (7) monté sur un chariot (11) coulisse sur le support (6). Un système motorisé (13) agit en levier sur le support (6) par l'intermédiaire du bras (12) pour une action en rotation.

Selon un mode particulier de réalisation, le plateau (2), dispose d'un système de montée motorisé permettant de relever la position des membres inférieurs.

Le dispositif selon l'invention est particulièrement destiné aux échocardiographies de stress à l'effort ou pharmacologiques, aux échocardiographies de repos, aux échocardiographies transoesophagiennes, aux angiologies et aux examens de repos.

## Revendications

1. Dispositif de plateau d'examen polyvalent, adapté à l'échocardiographie de stress à l'effort ou pharmacologique, à l'échocardiographie de repos, à l'échocardiographie transoesophagienne, à l'angiologie et aux examens de repos, permettant de passer dans un temps très court, d'une séquence d'examen à une autre séquence d'examen, de la manière la plus rapide possible et dans des conditions de confort, de rapidité et de qualité d'examens améliorées, une partie du plateau (2) destinée à recevoir le fessier et les membres inférieurs comportant un support (6) permettant la réception d'un ergomètre (7) et de ses accessoires, ce support prenant appui sur un axe transversal (8) monté sur un châssis du plateau (2) et situé à son extrémité, et un support de boîtiers de commandes (17) fixé sur le châssis du plateau (2), le dispositif étant **caractérisé en ce qu'**il comporte:
sur une partie têtière (1) : un orifice (3) de forme oblongue adapté au calage de la tête et pouvant être comblé par une pièce de forme identique, côté cœur, un support de forme cylindrique (4) à trois positions, sur la partie destinée à recevoir le fessier et
les membres inférieurs du plateau (2), un appui (5) composé de deux cylindres montés verticalement et inclinés vers l'extérieur, cet appui étant solidaire du châssis fixe du plateau (2) pour sa synchronisation lors de son inclinaison, un appui fessier (9) de forme rectangulaire et bombée sur le dessus, une poignée latérale réversible (10) fixée sur le côté droit du châssis du plateau (2).

2. Dispositif selon la revendication 1 **caractérisé en ce que** le support de l'ergomètre avec ses accessoires (14) est monté sur un axe libre (8) par l'intermédiaire d'un bras (12) de façon que ce support se situe au dessus du plateau (2), l'ergomètre (7) étant monté sur un chariot (11).

3. Dispositif selon les revendications 1 et 2 **caractérisé en ce que** le chariot (11) coulisse sur le support (6).

4. Dispositif selon les revendications 1 à 3 **caractérisé en ce qu'**un système motorisé (13) agit en levier sur le support (6) par l'intermédiaire du bras (12) pour une action en rotation de l'ergomètre (7).

5. Dispositif selon la revendication 1 **caractérisé en ce que** l'appui fessier (9) est escamotable manuellement ou par système motorisé.

6. Dispositif selon la revendication 1 **caractérisé en ce que** la poignée latérale (10) peut servir de support d'avant bras (15) par rotation de la poignée (10).

7. Dispositif selon les revendications 1 et 6 **caractérisé en ce que** le support de l'avant bras (15) dispose d'une attache (16) permettant d'immobiliser l'avant bras.

8. Dispositif selon la revendication 1 **caractérisé en ce que** le plateau (2) dispose d'un système de montée motorisé permettant de relever la position des membres inférieurs.

## Patentansprüche

1. Mehrzweck-Untersuchungstisch, für die Stress-Echokardiographie unter Belastung oder pharmakologisch angepasst, Echokardiographie im Ruhezustand, für die transösophagiale Echokardiographie, für die Angiologie und Untersuchungen im Ruhezustand, so kann in sehr kurzer Zeit von einer Untersuchungssequenz zu einer anderen Untersuchungssequenz gewechtselt werden, besonders schnell und komfortabel, und bei bester Untersuchungsqualität. Der Teil des Tisches (2), der dazu dient, das Gesäß und die unteren Gliedmaßen zum halten, verfügt über eine Halterung (6) für den Einsatz eines Fahrrad - Ergometer (7) samt Zubehör die auf einer Querachse (8) des Tisches, an einem Ende des Rahmens (2) befestigt ist, sowie eine Halterung für eine Steuerungselement (17), ebenfalls am Tischrahmen (2) angebracht, welches durch seine Bestandteile charakterisiert wird
Auf Kopfende (1) eine längliche Öffnung (3) angepasst an die Form/Neigung des Kopfes und mit einem entsprechend geformten Gegenstück zu Verschliessen. Herzseitig, eine zylindrische Halterung (4) mit drei Stellpositionen.
Auf dem Teil des Tisch (2) welcher dazu dient, das Gesäß und die unteren Gliedmaßen zu lagern, eine Stütze (5) bestehend aus zwei Zylindern, vertikal angebracht und nach aussen geneigt, welche fest mit dem eigentlichen Rahmen des Tisch (2) verbunden ist, für seine Synchronisation beim Kippen und sowie eine Gesäßstütze (9) rechteckig und gewölbt, und ein umkehrbarer Seitengriff (10) an der rechten Seite des Tischrahmens befestigt.

2. Mehrzweck-Untersuchungstisch nach Anspruch 1, dadurch charakterisiert, dass die Halterung (6) für den Fahrrad - Ergometer (7), samt ihres Zubehörs (14) auf einer freien Achse (8) montiert ist, mittels eines Arm (12), so, dass sich diese Halterung über dem Tisch (2) befindet, mit dem Fahrrad - Ergometer (7) auf einem Wagen (11) montiert.

3. Mehrzweck-Untersuchungtisch nach Anspruch 1 und 2, dadurch charakterisiert, dass der Wagen (11) auf der Halterung (6) scheibt.

4. Mehrzweck-Untersuchungtisch nach Ansprüche 1 bis 3 dadurch charakterisiert, dass ein motorisiertes System (13) dazu dient,die Halterung (6) anzuheben, mittels des Arms (12) wodurch das Fahrrad-Ergometer (7) gedreht werden kann.

5. Mehrzweck-Untersuchungtisch nach Anspruch 1 dadurch charakterisiert, dass die Gesäßstütze (9) manuell oder motorisch versenkbar ist.

6. Mehrzweck-Untersuchungtisch nach Anspruch 1 dadurch charakterisiert, dass der Seitengriff (10) als Unterarmstütze (15) dienen kann, wenn er umgedreht ist.

7. Mehrzweck-Untersuchungtisch nach Ansprüche 1 bis 6 dadurch charakterisiert, dass die Unterarmstütze (15) hat eine Armbindung (16) um den Unterarm ruhig zu stellen.

8. Mehrzweck-Untersuchungtisch nach Anspruch 1 dadurch charakterisiert, dass der Tisch (2) über ein motorisches Hebersystem zum anheben verfügt, welches es erlaubt, die Position der unteren Gliedmassen zu erhören.

## Claims

1. Multi-purpose examination table suitable for stress or pharmacological echocardiography , for echocardiography at rest, for transesophageal echocardiography, for angiology and for examinations at rest, allowing to pass in a very short time, from one examination sequence to another examination sequence, as quickly as possible and under improved conditions of comfort, speed and examination quality. A part of the table (2) intended to receive the buttocks and the lower limbs, comprises a support (6) for receiving an ergometer (7) and the accessories thereof. This support bears on a transverse shaft (8) mounted on the frame of the table (2) situated at the end thereof and a control box support (17) fixed to the frame of the table (2), the device being **characterized in that** it comprises:
on the headrest part (1), an oblong orifice (3) suitable for securing the head and being able to be filled by a component of identical shape and towards the heart, a cylindrical support (4) with three positions and
on the part of the table (2) intended to receive the buttocks and the lower limbs, a rest (5) composed of two cylinders mounted vertically and inclined towards the outside, this rest being rigidly connected to the fixed frame of the table (2) for its synchronization during its inclination, a buttocks rest (9) of rectangular shape and curved and a reversible lateral handle (10) fixed to the right-hand side of the frame of the table (2).

2. Device according to claim 1 **characterized in that** the support of the ergometer (7) and the accessories thereof (14) are mounted on a free axis (8) by means of an arm (12) so that this support is situated above the table (2), the ergometer (7) being mounted on a case (11).

3. Device according to claims 1 and 2 **characterised in that** the case (11) slides on the support (6).

4. Device according to claims 1 to 3 **characterized in that** a motorized system operates as a lever on the support (6) via the arm (12) for an action in rotation of the ergometer (7).

5. Device according to claim 1 **characterised in that** the buttocks rest (9) is retractable manually or by a motorised system.

6. Device according to claim 1 **characterised in that** the lateral handle (10) can be used as a support for the forearm by rotating the handle (10).

7. Device according to claims 1 to 6 **characterized in that** the forearm support (15) has a clip (16) allowing the forearm (15) to be immobilized .

8. Device according to claim 1 **characterised in that** the table (2) has a lifting system allowing for raising the lower limbs.
